# EUROPEAN PATENT SPECIFICATION

(11) **EP 4 266 043 B1**
(45) Date of publication and mention of the grant of the patent: **05.08.2026**
(21) Application number: 21911348.7
(22) Date of filing: 10.12.2021
(51) Int. Cl.: B21C 51/00, C21D 9/46, G01N 27/80

(54) **ELECTROMAGNETIC INSPECTION DEVICE CAPABLE OF CALIBRATION**
ELEKTROMAGNETISCHES PRÜFGERÄT MIT KALIBRIERFUNKTION
APPAREIL D'INSPECTION ÉLECTROMAGNÉTIQUE APTE À L'ÉTALONNAGE

(30) Priority: 21.12.2020 KR 20200179341
(43) Date of publication of application: 25.10.2023
(73) Proprietor: POSCO Co., Ltd, Pohang-si, Gyeongsangbuk-do 37859 (KR)
(72) Inventor: LEE, Ju-Seung, Pohang-si, Gyeongsangbuk-do 37877 (KR); KOH, Seong-Ung, Pohang-si, Gyeongsangbuk-do 37877 (KR); PARK, Hyoung-Kuk, Pohang-si, Gyeongsangbuk-do 37877 (KR)
(74) Representative: Nederlandsch Octrooibureau
(86) International application number: PCT/KR2021/018740
(87) International publication number: WO 2022/139281

(56) References cited:
- JP-A- 2019 042 807
- JP-A- 2019 042 807
- JP-A- H11 326 296
- JP-B2- 6 285 893
- KR-A- 20060 063 430
- KR-A- 20130 126 962
- KR-B1- 100 949 316
- KR-B1- 101 767 774

## Description

### Technical Field

The present disclosure relates to an inspection device used for sensor calibration.

### Background Art

As a general hardness measurement method, a destructive method of measuring hardness strength according to the shape thereof by applying force with a specific load to the surface of the object to be measured is widely used. This method is a method of calculating the hardness value by measuring the shape of the section to be destroyed. However, in the case of a generally used steel plate, the length is several meters to several tens of meters, and the width is several meters, and in order to measure the hardness of the entire area of the steel sheet, it is practically impossible to measure the hardness of the entire section using related art methods.

As in Patent Document 1 or Patent Document 2, a technology has been developed to measure the characteristics of a steel sheet by generating eddy currents in the steel sheet through a coil to which AC power is applied without contact, but there is a problem in which accuracy is insufficient and the plate-like material cannot be measured quickly and accurately.

On the other hand, Patent Document 2 discloses the configurations in which an eddy current is generated in the steel sheet through a coil to which AC power is applied, a sensor for measuring the characteristics of the steel sheet is used while calibrating the sensitivity with a correction steel sheet installed, and the test piece is inserted or contains artificial defects caused by arc strikes. In the case of such a correction steel sheet, there may be a problem that it is insufficient to calibrate precise sensitivity.

### [Prior art literature]

### [Patent Literature]

(Patent Document 1) JP 2000-227421 A
(Patent Document 2) JP 2019-042807 A

### Summary of Invention

### Technical Problem

An aspect of the present disclosure is to provide an electromagnetic inspection device including a calibration steel sheet for quickly and accurately calibrating sensitivity of a sensor in hardness measurement in which a sudden signal change does not occur.

### Solution to Problem

In order to achieve the above object, the present disclosure provides an electromagnetic inspection device as defined in claim

In an embodiment, the second portion may have a hardness higher than the first portion by 50 Hv or more, and the first portion and the second portion may be formed extending in a long side direction.

In an embodiment, the second portion may have a hardness of 250 Hv or more.

In an embodiment, the steel sheet for sensor calibration may include a third portion having the third hardness, wherein the third hardness may be greater than the first hardness and less than the second hardness, the third portion may be disposed in a different position to those of the first and second portions in the short side direction, and the third portion may have a phase transformed from austenite grains.

In an embodiment, the calibration unit may perform calibration by converting the measured value measured by the electromagnetic sensor into hardness, the same as the hardness of the measured portion.

In an embodiment, the steel sheet to be measured and the steel sheet for sensor calibration may have the same length and thickness.

In an embodiment, a measurement area in which the steel sheet to be measured is disposed and a calibration area in which the steel sheet for sensor calibration is disposed may be disposed side by side.

According to an aspect of the present disclosure (not part of the claimed invention), method of manufacturing a calibration steel sheet, as a method of manufacturing a steel sheet for sensor calibration, includes a heating operation of heating the steel sheet in a heating furnace; a rolling operation of rolling a heated steel sheet with a rolling roll; and a cooling operation of cooling a rolled steel sheet, wherein in the cooling operation, the steel sheet includes a first portion and a second portion in different positions in a width direction of the steel sheet, and the cooling rates of the first portion and the second portion are different.

In an example, in the cooling operation, an adjustment of the cooling rate may be performed by adjusting a cooling quantity of a corresponding portion.

In an example, in the cooling operation, the cooling rate of the second portion may be higher than the first portion.

In an example, in the cooling operation, the cooling rate may be controlled by adjusting an amount of cooling water supplied in a width direction and a longitudinal direction of the steel sheet.

### Advantageous Effects of Invention

Through the configuration, the present disclosure may provide an electromagnetic inspection device comprising a calibration steel sheet for calibrating the sensitivity of a sensor in hardness measurement in which a sudden signal change does not occur.

### Brief Description of Drawings

FIG. 1 is a cross-sectional image of a surface layer of a steel sheet, in which (a) is a cross-sectional image of a high-hardness portion obtained by mold powder, and (b) is a cross-sectional image of a high-hardness portion obtained by local supercooling.
FIG. 2 is a cross-sectional image of the surface layer of the steel sheet, in which (a) is a cross-sectional image of a high-hardness portion formed by arcing, and (b) is a cross-sectional image of a high-hardness portion formed by local supercooling.
FIG. 3 is a process flow chart for manufacturing a calibration steel sheet according to the present disclosure.
FIG. 4 is a schematic diagram of a calibration steel sheet according to the present disclosure.
FIG. 5 is a schematic diagram of a cooling process in an embodiment.
FIG. 6 is a schematic diagram of a cooling process in another embodiment.
FIG. 7 is a schematic diagram of an electromagnetic inspection device capable of calibration according to the present invention.
FIG. 8 is a graph of hardness taken in the width direction of the calibration steel sheet of FIG. 7.
FIG. 9 is a graph of a signal taken in the width direction of the calibration steel sheet illustrated in FIG. 7.
FIG. 10 is a signal graph obtained from each electromagnetic sensor in FIG. 7.
FIG. 11 is a graph obtained by calibrating the signal graph of FIG. 10 using a calibration unit.
FIG. 12 is a hardness graph obtained when the same steel plate is inspected when calibration is performed with a calibration steel sheet according to an embodiment of the present disclosure and when calibration is performed with a calibration steel sheet according to a comparative example.

### Description of Reference Characters

1: electromagnetic inspection device 10: water supply facility
20: roll 100: sensor body
110: electromagnetic sensor 120: guide bar
130: support portion
P: steel sheet to be measured CP: calibration steel sheet
Z1: measurement area Z2: calibration area

### Best Mode for Invention

Hereinafter, detailed embodiments of the present disclosure will be described with reference to the accompanying drawings.

The thick plate may be used as an oil pipe or gas pipe, and in this case, rigidity and sour resistance are required so that there are no problems caused by materials passing through the inside. Generally, Thermo-Mechanical Control Process Steel (TMCP) is used as a thick plate with rigidity and sour resistance.

In such a thick plate, in the case in which the hardness of the thick plate is high hardness (250Hv) higher than the standard hardness of 200Hv, hydrogen induced cracking (HIC) may occur in which such a high hardness portion reacts with H2S under high-pressure conditions to generate cracks and destroy the pipe. This content is also disclosed in "DNV-OS-F101 (Submarine Pipeline System)" or "API 5L Specification for Line Pipe".

Therefore, it is necessary to accurately determine the hardness of the subsurface portion of the thick plate to prevent defects that may occur in the future. In particular, in the case of occurrence of an accident in a raw material transport pipe, complex problems such as an environmental pollution as well as human/material loss significantly occur, and thus, accurate hardness measurement is required for the entire area of high-strength, high-corrosion-resistant steel thick plate.

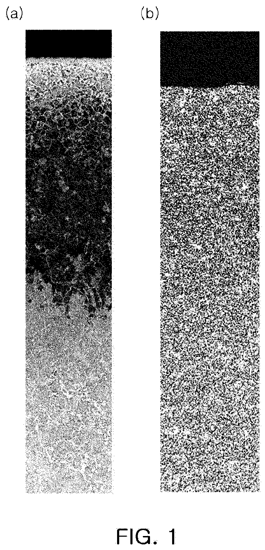

There may be several factors that cause high hardness in thick plates, but there are two well-known factors, carburization and local supercooling, and FIG. 1 illustrates a cross-sectional image of a high hardness portion generated by these factors. FIG. 1(a) illustrates a cross-sectional image of a high-hardness portion generated by carburization, and FIG. 1(b) illustrates a cross-sectional image of a high-hardness portion generated by local supercooling.

As illustrated in FIGS. 1 (a) and (b), in the case of carburization, mold powder (C: 0.1 wt% or more) remains on the surface layer of the slab, and the boundary of the defect structure is clear, and the high hardness portion has a different composition from the other portions. Therefore, it may be easily detected in an electromagnetic inspection device. On the other hand, in the case of local supercooling, it has the same composition as the material, but the phase transformed from austenite by subcooling is different, and since the temperature gradient occurs continuously, the boundary of the defect structure is unclear, which has a problem in that it is difficult to accurately detect flaws in an electromagnetic inspection device. Therefore, it is necessary to develop an inspection device capable of determining defects that are relatively difficult to measure.

In the case of an electromagnetic inspection device, the electromagnetic inspection device is a device in which an electric field or magnetic field is applied to a steel plate, and a signal changed by the material characteristics of the steel plate is sensed, or an additional sensor is disposed to measure current/voltage/magnetic field strength and the like generated by the material characteristics of the steel plate, thereby inspecting characteristics of the steel plate.

Hardness is also a kind of material property, for example, when inspecting with an eddy current measuring device and when the hardness value increases, the measured signal value becomes smaller (an increase in energy loss) due to the characteristic that the width of the hysteresis curve increases in the B-H curve, and by using this, the hardness value of the material may be measured. However, since there are various factors affecting the signal to be measured, it is difficult to know whether the measured signal value is changed by hardness or by other factors such as residual stress, potential, and residual magnetization. This situation is the same even in the case of a leakage flux the signal to be measured, it is difficult to know whether the measured signal value is changed by hardness or by other factors such as residual stress, potential, and residual magnetization. This situation is the same even in the case of a leakage flux measuring device other than an eddy current measuring device.

On the other hand, in the case of an electromagnetic inspection device, measurement is performed through a plurality of sensors for rapid inspection. In the case of respective sensors, even if the same object is measured, since the sensing values are different, it is necessary to calibrate this, and at this time, the calibration work usually utilizes a material of which exact value is known.

Usually, a small sample of which the exact value is known is prepared, and the sensor passes through the sample to calibrate the measured signal value to a known value, and as mentioned above, the hardness of the sample is accurately known, but the information for other factors is not accurate, and therefore, it cannot be said that the conditions are the same as the measurement target, and this makes calibration difficult.

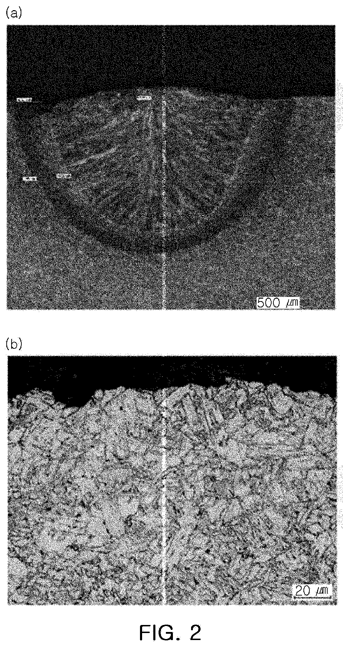

Alternatively, a high-hardness part is made by creating artificial defects in the steel sheet. In order to create a high-hardness part, the case of arcing - in which a material is melted and then solidified to form high hardness - is disclosed in Patent Document 2. FIG. 2(a) illustrates a high-hardness portion by re-solidification, it is the same in terms of high hardness, but the high-hardness portion has a re-solidified solidification structure. However, considering that the crystal grains are maintained in the case of local supercooling, and that the electromagnetic signal is also affected by the grain boundaries, it can be seen that electromagnetic signals are not equally generated even with the same hardness.

Therefore, in the case of simply constructing a calibration plate based on the hardness value, since calibration is performed based on defects that are different from actual defects, the signal value is affected by factors other than hardness, and thus, accurate measurement is not possible, and this makes inspection of defects due to local supercooling more difficult.

Although the present disclosure is described mainly on the eddy current inspection device in an embodiment, the electromagnetic inspection device is not limited to the eddy current inspection device and may be applied to various electromagnetic inspection devices.

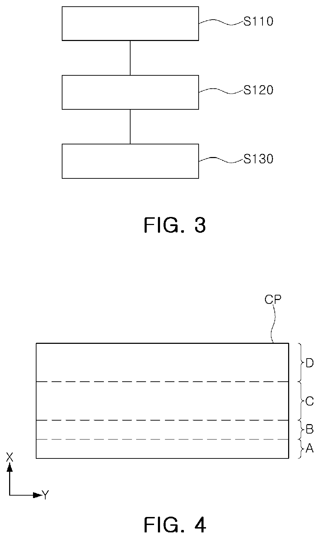

FIG. 3 illustrates a flow chart of a method of manufacturing a calibration steel sheet according to the present disclosure, and FIG. 4 illustrates a plan view of a calibration steel sheet (CP) according to the present disclosure.

The present disclosure provides a calibration steel sheet capable of calibrating an electromagnetic sensor to accurately measure a high-hardness steel plate with an electromagnetic sensor, and a method of manufacturing the same, and has the basic concept to create a local supercooling defect that is difficult to sense on a calibration steel sheet and to calibrate an electromagnetic sensor based thereon. In addition, the factor that may affect the signal value of the electromagnetic sensor in the calibration steel sheet is made the same as the steel sheet to be measured, such that the relationships between the reference value obtained by actual measurement and the signal value of the sensor are easily and quickly matched, thereby enabling rapid and accurate measurement without deviation between sensors.

FIG. 3 illustrates a method of manufacturing a calibration steel sheet (CP) according to the present disclosure. In an embodiment of the present disclosure, the calibration steel sheet (CP) has the same composition as the steel sheet to be measured (P), is manufactured to have the same size (thickness/width/length), and is produced through a heating operation (S110), a rolling operation (S120), and a cooling operation (S130) . The heating operation (S110) and the rolling operation (S120) are basically the same as the manufacturing process of the steel sheet P to be measured. That is, after heating in a heating furnace to make a heating slab, it is rolled in a rolling mill to have the same size (thickness/width/length) as the steel sheet P to be measured.

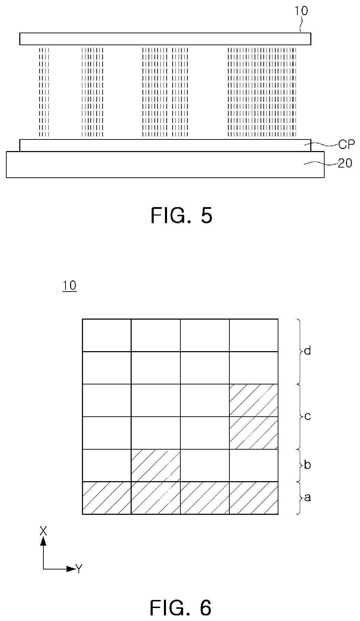

After that, it is cooled in the cooling operation (S130), and the cooling state is illustrated in FIG. 5. As illustrated in FIG. 5, in the cooling operation (S130), the calibration steel sheet CP is transported by a roll 20 and cooled by water sprayed from a water supply facility 10. When viewed from the left side of FIG. 5, the calibration steel sheet CP is cooled while varying the amount of water sprayed from the water supply facility 10 in a plurality of areas A, B, C, and D. The steel sheet having an austenite phase in the rolling operation (S120) is cooled at different cooling rates in the cooling operation (S130), thereby having different phases. At this time, the areas of local supercooling, for example, areas B and D, have a lot of bainite and become relatively hard, and in the case of area A, the cooling rate is low, resulting in low hardness. Any equipment may be applied as the water supply facility 10 as long as it is a facility capable of adjusting the water supply amount in the width direction (X direction) of the steel plate, and for example, a facility such as KR 10-1767774 may be applied.

When cooled by the equipment as illustrated in FIG. 5 in this manner, a calibration steel sheet (CP) as illustrated in FIG. 4 is manufactured. After cooling is completed, the calibration steel sheet CP is moved to a calibration area Z2 of the electromagnetic inspection device 1 to be described later, and is demagnetized by a demagnetization facility.

In the case of the calibration steel sheet (CP) of the present disclosure, it is a steel sheet having a short side (width direction, X direction in FIG. 4) and a long side (longitudinal direction, Y direction in FIG. 4), and includes regions having different hardnesses in the width direction (X direction) of the steel sheet. As described above, respective areas A, B, C, and D are distinguished in the width direction, and have the same length as the length of the steel plate in the longitudinal direction.

Respective regions have the same composition, but are cooled at different cooling rates, and therefore, the fractions of the phases transformed in the austenite grains are different from each other. The higher the higher hardness region is, the higher the bainite fraction may be, and the lower the lower hardness region is, the higher the ferrite fraction may be.

Since the calibration steel sheet (CP) of the present disclosure has different hardnesses in the width direction, a plurality of hardnesses required for calibration may be provided. In addition, since it is manufactured with the same composition and size as the steel sheet P to be measured, it may be magnetized/demagnetized in the same manner during magnetization or demagnetization. That is, in that the degree of magnetization/demagnetization does not vary due to the size, factors to be considered when measuring the steel sheet P to be measured and when measuring the calibration steel sheet CP may be reduced, and accurate calibration may be provided.

In addition, since the heating and rolling operations (S110 and S120) go through the same operations as the steel sheet P to be measure, the factors affecting magnetization in the heating and rolling operations (S110 and S120), for example, the residual stress corresponds to the same or at least to a similar level, and this guarantees that the magnitude of the signal that changes as the hardness changes in the calibration steel sheet (CP) may also be obtained equally in the steel sheet (P) to be measured.

It is advantageous for calibration to have a hardness difference of 50 Hv or more between the low hardness region (A) and the high hardness region (B or D), and it is desirable that the high hardness region (B or D) is 250 Hv or more with the possibility of hydrogen induced cracking (HIC) for accurate inspection after calibration.

It is also possible to have a medium hardness region (C) between the low hardness region (A) and the high hardness region (B or D), and accurate calibration may be possible by providing a plurality of different hardness regions (A, B, C, and D) including the medium hardness region (C) based on at least three points.

On the other hand, FIG. 6 illustrates another cooling method of the cooling operation (S130). As illustrated in FIG. 6, in the case of a facility in which a water supply device 10 may adjust the water supply amount not only in the width direction (X direction) but also in the longitudinal direction (Y direction), the water supply amount is adjusted in both the width direction and the longitudinal direction, and a calibration steel sheet (CP) in which the cooling rate is more precisely controlled in the width direction (X direction) of the steel sheet may be manufactured, and this steel sheet enables precise calibration in a calibration-capable electromagnetic inspection device.

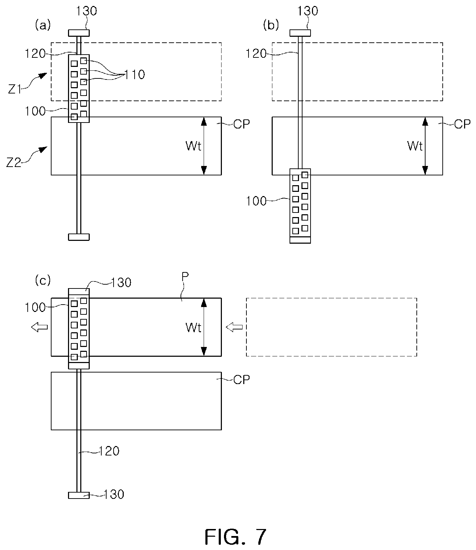

FIG. 7 illustrates an electromagnetic inspection device 1 capable of calibration according to the present invention. FIG. 7 (a) is a schematic diagram of a state in which calibration starts in the electromagnetic inspection device 1, FIG. 7 (b) is a schematic diagram of a state in which calibration is being completed in the electromagnetic inspection device 1, and FIG. 7(c) is a schematic diagram of a state in which the electromagnetic inspection device 1 inspects the steel sheet P to be measured after completion of calibration.

As illustrated in FIG. 7, the electromagnetic inspection device 1 according to the present invention is an electromagnetic sensor calibration device 1 including a sensor body 100 including a plurality of electromagnetic sensors 110 disposed in the width direction of the steel sheet P to be measured; a moving unit connected to the sensor body 100 and moving the sensor body 100; a calibration steel sheet (CP) disposed in a different position to that of the steel sheet to be measured (P); and a calibration unit connected to the electromagnetic sensor 110.

The sensor body 100 has a configuration in which the plurality of electromagnetic sensors 110 are arranged in a plurality of rows taken in the width direction (X direction) of the steel sheet. In this embodiment, the sensor array is configured to measure the width of the steel sheet at one time, but is not limited thereto, and it is also possible to measure while being moved in the width direction of the steel sheet by the moving unit.

The electromagnetic sensor 110 measures hardness using an electromagnetic method, and may be, for example, an eddy current measurement sensor or a leakage flux measurement sensor, and in this embodiment, is an eddy current measurement sensor as mentioned above.

The moving unit is configured to move the sensor body 100 in the X direction or in the X and Y directions while maintaining the same distance with respect to the surface of the steel plate, and may include a linear movement unit (not illustrated) for linearly moving the sensor body 100 between a support portion 130 and a guide bar 120 on both sides and the sensor body 100 and the guide bar 120. In the case of the moving unit, as long as horizontal movement is possible, various structures may be applied.

In the case of the electromagnetic inspection device 1 according to an embodiment of the present disclosure, the sensor body 100 is moved to a measurement area Z1 and a calibration area Z2 by the moving unit. The measurement area Z1 and the calibration area Z2 may be located side by side.

The calibration steel sheet CP is disposed in the calibration area Z2, and the steel sheet P to be measured may be supplied to and discharged from the measurement area Z1. When inspecting the steel sheet P to be measured in the measurement area Z1, the steel sheet P to be measured may be moved by a conveying unit such as a roller while the sensor body 100 is fixed. Alternatively, when the steel sheet P to be measured is supplied to the measurement area Z1, it is possible to inspect the steel sheet while the sensor body 100 is moved in the Y direction by the moving unit, and it is also possible to test by a combination of the two.
In this embodiment, in the case of the electromagnetic inspection device 1, in the calibration area Z2, the moving unit may move the sensor body 100 in the width direction at least as much as the width W of the steel sheet P to be measured or the calibration steel plate CP. Since the width of the calibration steel sheet CP is the same as the width W of the steel plate P to be measured, in order for all electromagnetic sensors 110 to inspect the entire regions of the calibration steel sheet (CP) in the width direction, the moving unit should move the sensor body 100 by at least the sum of the width of the calibration steel sheet CP and a maximum distance between the electromagnetic sensors. The maximum distance between the electromagnetic sensors means the distance between the electromagnetic sensors on both ends in the X direction. This appearance is illustrated in FIGS. 7(a) and 7(b).

In a case in which the calibration area (Z2) is the same as the measurement area (Z1), that is, even when the calibration steel sheet CP is supplied/discharged to the same area as the steel sheet P to be measured, the moving unit should measure while moving the sensor body in the width direction of the steel sheet by the sum of the width (W) and the maximum distance between the electromagnetic sensors. In this case, moving may mean relative motion.

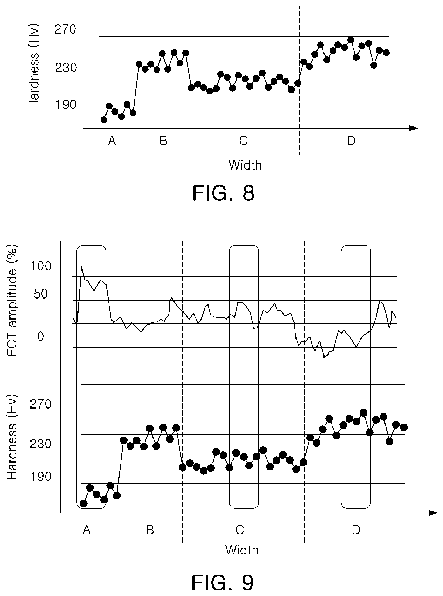

The calibration method will be described later with reference to FIGS. 8 to 11, and after the calibration of the electromagnetic sensor 110 is completed, the moving unit returns the sensor body 100 to the measurement area Z1, and after returning, the measured steel sheet P supplied to the measurement area Z1 is inspected. By calibration, respective electromagnetic sensors 110 may provide the same hardness measurement value even if they are sensors that measure different measurement values at the same hardness, and therefore, accurate inspection may be performed.

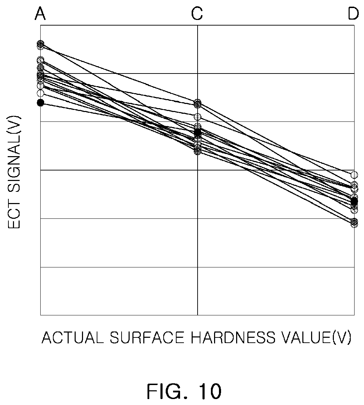

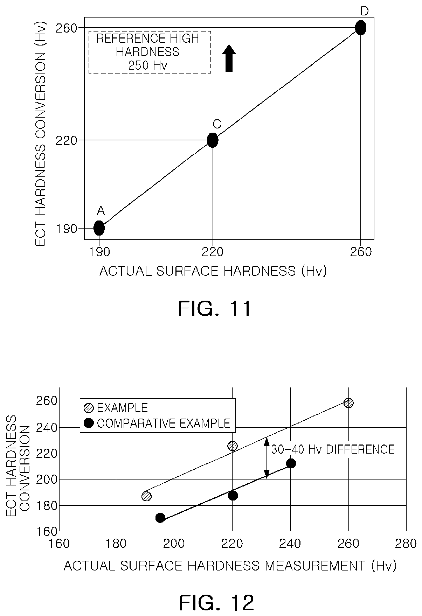

FIG. 8 to 11 illustrate graphs according to a calibration method of an electromagnetic inspection device capable of being calibrated. FIG. 8 illustrates a graph of hardness in the actual width direction of the calibration steel sheet, FIG. 9 illustrates a signal graph and a selection area according to the width direction of the calibration steel sheet, FIG. 10 illustrates measured values of each electromagnetic sensor in each selected area, and FIG. 11 illustrates a graph of measured hardness and actual hardness corrected by calibration.

As illustrated in FIG. 8, after the calibration steel sheet (CP) is manufactured, the actual hardness value is measured by the Leeb test, and the hardness is illustrated in FIG. 8. As illustrated in FIG. 8, area A has low hardness, areas B and D have high hardness, and area C has medium hardness between A and B.

The calibration unit connected to the electromagnetic sensor 110 selects a plurality of areas having different hardnesses. For example, as illustrated in FIG. 9, three regions of low hardness, medium hardness, and high hardness are selected, and when the electromagnetic sensor passes the corresponding portion, a signal value is measured, and a graph of signals measured in this manner is illustrated in FIG. 10. As illustrated in FIG. 10, since respective sensors are not exactly the same even if the same portion is measured, the signal values measured by respective sensors are inevitably different. In the present disclosure, the signal value is converted to match the actual hardness value through a calibration unit connected to the sensor, and the conversion formula or correspondence relationship for each sensor is set so that the actual hardness and the converted hardness value always coincide. In this manner, by setting the conversion formula or correspondence relationship for each sensor, calibration of each sensor is completed, and the sensor body 100 including the sensor of which calibration has been completed is sent to the measurement area Z1 to measure the steel sheet P to be measured.

As mentioned above, the steel sheet including the region of high hardness, for example, 250 Hv by the sensor 110 is found by the electromagnetic inspection device 1, and after removing the high hardness region through an additional process or undergoing heat treatment to lower the hardness, it is supplied to consumers as a product. Therefore, it is possible to provide a thick plate material free from hydrogen-induced cracks.

FIG. 12 illustrates a graph when the same steel sheet is measured with a sensor calibrated using the calibration steel sheet according to the present disclosure (Example) and a sensor calibrated using a calibration sample of the related art (Comparative Example).

As illustrated in FIG. 12, even if calibration is performed using a calibration sample of the related art, there is no difference in tendency from that of the examples, but a problem occurs that the measured hardness and the actual hardness do not match. However, in the embodiment of the present disclosure, the above problem does not occur because the measured hardness and the actual hardness may be matched by correcting only the difference with respective sensors. In particular, in the case of the comparative example, the reason for the difference between the measured hardness and the actual hardness should be analyzed each time, and in the case of the embodiment, since the basic conditions are the same for the steel sheet for measurement and the calibration steel sheet, and thus, there is no need for analysis for this reason, and it is possible to easily and quickly calibrate the steel sheet with respect to various steel sheets.

In the present disclosure, a calibration steel sheet may be manufactured by changing only the cooling operation in the actual manufacturing process, and in the case of utilizing this, there is no need to consider a number of factors, which is advantageous for continuously manufacturing and calibration steel sheets.

In the above, the embodiment of the present disclosure has been mainly described, but the present disclosure is not limited to the above embodiment and may be variously modified and implemented. The scope of protection is defined by the appended claims .

## Claims

1. An electromagnetic inspection device (1) capable of calibration comprising:
a sensor body (100) including a plurality of electromagnetic sensors (110) disposed in a width direction of a steel sheet to be measured (P);
a moving unit connected to the sensor body and configured to move the sensor body; and
a calibration steel sheet (CP) disposed in a different position to that of the steel sheet to be measured,
wherein the calibration steel sheet having a long side and a short side and configured to be used for correcting a hardness measurement sensor, comprising: a first portion having a first hardness and a second portion having a second hardness higher than the first hardness,
wherein the first portion and the second portion are disposed in different positions in a short side direction, and
the first portion and the second portion have transformed phases while maintaining the grain structure from austenite grains of the same composition,
wherein the calibration steel sheet has the same material and width as the steel sheet to be measured, and
on the calibration steel sheet, the moving unit is configured to have a movement distance equal to or greater than a sum of a maximum distance between the plurality of electromagnetic sensors and a width of the calibration steel sheet,
and the device further comprising a calibration unit (140) connected to the plurality of electromagnetic sensors,
wherein the calibration unit is configured to calibrate the plurality of electromagnetic sensors based on a measurement value obtained by measuring the first portion and a measurement value obtained by measuring the second portion with the plurality of electromagnetic sensors.

2. The electromagnetic inspection device capable of calibration of claim 1, wherein the second portion has a hardness higher than the first portion by 50 Hv or more, and
the first portion and the second portion are formed extending in a long side direction.

3. The electromagnetic inspection device capable of calibration of claim 1, wherein the second portion has a hardness of 250Hv or more.

4. The electromagnetic inspection device capable of calibration of claim 3, comprising a third portion having a third hardness,
wherein the third hardness is greater than the first hardness and less than the second hardness, the third portion is disposed in a different position to those of the first and second portions in the short side direction, and
the third portion has a phase transformed from austenite grains.

5. The electromagnetic inspection device capable of calibration of claim 1, wherein when the calibration unit converts the measured value measured by the electromagnetic sensors into hardness,
the calibration unit performs correction, to be converted equally to hardness of the measured portion.

6. The electromagnetic inspection device capable of calibration of claim 1, wherein the steel sheet to be measured and the calibration steel sheet have the same length and thickness.

7. The electromagnetic inspection device capable of calibration of claim 1, wherein a measurement area (Z1) in which the steel sheet to be measured is disposed and a calibration area (Z2) in which the calibration steel sheet is disposed are disposed side by side.

## Patentansprüche

1. Elektromagnetische Inspektionsvorrichtung (1), die zur Kalibrierung fähig ist und Folgendes umfasst:
einen Sensorkörper (100), der eine Vielzahl elektromagnetischer Sensoren (110) enthält, die in Breitenrichtung eines zu messenden Stahlblechs (P) angeordnet sind;
eine Bewegungseinheit, die mit dem Sensorkörper verbunden und so konfiguriert ist, dass sie den Sensorkörper bewegt; und
ein Kalibrierungsstahlblech (CP), das an einer anderen Position als das zu messende Stahlblech angeordnet ist,
wobei das Kalibrierungsstahlblech eine lange Seite und eine kurze Seite aufweist und so konfiguriert ist, dass es zur Korrektur eines Härtemesssensors verwendet werden kann, und Folgendes umfasst: einen ersten Teil mit einer ersten Härte und einen zweiten Teil mit einer zweiten Härte, die höher als die erste Härte ist,
wobei der erste Teil und der zweite Teil an unterschiedlichen Positionen in Richtung der kurzen Seite angeordnet sind, und
wobei der erste Teil und der zweite Teil transformierte Phasen aufweisen, während die Kornstruktur aus Austenitkörnern derselben Zusammensetzung erhalten bleibt,
wobei das Kalibrierungsstahlblech das gleiche Material und die gleiche Breite wie das zu messende Stahlblech aufweist, und
wobei die Bewegungseinheit auf dem Kalibrierungsstahlblech so konfiguriert ist, dass ihre Bewegungsstrecke gleich oder größer als die Summe aus dem maximalen Abstand zwischen der Vielzahl elektromagnetischer Sensoren und der Breite des Kalibrierungsstahlblechs ist,
und die Vorrichtung ferner eine Kalibrierungseinheit (140) umfasst, die mit der Vielzahl elektromagnetischer Sensoren verbunden ist,
wobei die Kalibrierungseinheit so konfiguriert ist, dass sie die Vielzahl elektromagnetischer Sensoren basierend auf einem Messwert, der durch Messen des ersten Teils erhalten wird, und einem Messwert, der durch Messen des zweiten Teils mit der Vielzahl elektromagnetischer Sensoren erhalten wird, kalibriert.

2. Elektromagnetische Inspektionsvorrichtung, die zur Kalibrierung fähig ist, nach Anspruch 1, wobei der zweite Teil eine um 50 Hv oder mehr höhere Härte als der erste Teil aufweist, und
der erste Teil und der zweite Teil so ausgebildet sind, dass sie sich in Richtung der langen Seite erstrecken.

3. Elektromagnetische Inspektionsvorrichtung, die zur Kalibrierung fähig ist, nach Anspruch 1, wobei der zweite Teil eine Härte von 250 Hv oder mehr aufweist.

4. Elektromagnetische Inspektionsvorrichtung, die zur Kalibrierung fähig ist, nach Anspruch 3, die einen dritten Teil mit einer dritten Härte umfasst,
wobei die dritte Härte größer als die erste Härte und kleiner als die zweite Härte ist,
wobei der dritte Teil in Richtung der kurzen Seite an einer anderen Position als der erste und zweite Teil angeordnet ist, und
der dritte Teil eine Phase aufweist, die aus Austenitkörnern umgewandelt ist.

5. Elektromagnetische Inspektionsvorrichtung, die zur Kalibrierung fähig ist, nach Anspruch 1, wobei, wenn die Kalibrierungseinheit den von den elektromagnetischen Sensoren gemessenen Wert in Härte umwandelt,
die Kalibrierungseinheit eine Korrektur ausführt, die gleichmäßig in die Härte des gemessenen Teils umgewandelt wird.

6. Elektromagnetische Inspektionsvorrichtung, die zur Kalibrierung fähig ist, nach Anspruch 1, wobei das zu messende Stahlblech und das Kalibrierungsstahlblech die gleiche Länge und Dicke aufweisen.

7. Elektromagnetische Inspektionsvorrichtung, die zur Kalibrierung fähig ist, nach Anspruch 1, wobei ein Messbereich (Z1), in dem das zu messende Stahlblech angeordnet ist, und ein Kalibrierungsbereich (Z2), in dem das Kalibrierungsstahlblech angeordnet ist, nebeneinander angeordnet sind.

## Revendications

1. Dispositif d'inspection électromagnétique (1) capable d'étalonnage comprenant :
un corps de capteur (100) incluant une pluralité de capteurs électromagnétiques (110) disposés dans le sens de la largeur d'une tôle d'acier à mesurer (P) ;
une unité de déplacement connectée au corps de capteur et configurée pour déplacer le corps de capteur ; et
une tôle d'acier d'étalonnage (CP) disposée dans une position différente de celle de la tôle d'acier à mesurer,
où la tôle d'acier d'étalonnage ayant un côté long et un côté court et configurée pour être utilisée pour corriger un capteur de mesure de dureté, comprend: une première portion ayant une première dureté et une deuxième portion ayant une deuxième dureté supérieure à la première dureté,
où la première portion et la deuxième portion sont disposées dans des positions différentes dans le sens d'un côté court, et
la première portion et la deuxième portion subissent des transformations de phase tout en conservant la structure granulaire des grains d'austénite de même composition,
où la tôle d'acier d'étalonnage présente le même matériau et la même largeur que la tôle d'acier à mesurer, et
sur la tôle d'acier d'étalonnage, l'unité de déplacement est configurée pour avoir une distance de déplacement égale ou supérieure à une somme d'une distance maximale entre la pluralité de capteurs électromagnétiques et d'une largeur de la tôle d'acier d'étalonnage,
et le dispositif comprend en outre une unité d'étalonnage (140) connectée à la pluralité de capteurs électromagnétiques,
où l'unité d'étalonnage est configurée pour étalonner la pluralité de capteurs électromagnétiques à partir d'une valeur de mesure obtenue en mesurant la première portion et d'une valeur de mesure obtenue en mesurant la deuxième portion avec la pluralité de capteurs électromagnétiques.

2. Dispositif d'inspection électromagnétique capable d'étalonnage selon la revendication 1, où la deuxième portion a une dureté supérieure à la première portion de 50 HV ou plus, et la première portion et la deuxième portion sont formées par extension dans le sens d'un côté long.

3. Dispositif d'inspection électromagnétique capable d'étalonnage selon la revendication 1, où la deuxième portion a une dureté de 250 HV ou plus.

4. Dispositif d'inspection électromagnétique capable d'étalonnage selon la revendication 3, comprenant une troisième portion ayant une troisième dureté,
où la troisième dureté est supérieure à la première dureté et inférieure à la deuxième dureté,
la troisième portion est disposée dans une position différente de celles des première et deuxième portions dans le sens du côté court, et
la troisième portion présente une phase transformée à partir de grains d'austénite.

5. Dispositif d'inspection électromagnétique capable d'étalonnage selon la revendication 1, où lorsque l'unité d'étalonnage convertit la valeur mesurée par les capteurs électromagnétiques en dureté,
l'unité d'étalonnage effectue une correction qui sera également convertie en dureté de la portion mesurée.

6. Dispositif d'inspection électromagnétique capable d'étalonnage selon la revendication 1, où la tôle d'acier à mesurer et la tôle d'acier d'étalonnage ont la même longueur et la même épaisseur.

7. Dispositif d'inspection électromagnétique capable d'étalonnage selon la revendication 1, où une zone de mesure (Z1) dans laquelle la tôle d'acier à mesurer est disposée et une zone d'étalonnage (Z2) dans laquelle la tôle d'acier d'étalonnage est disposée sont disposées côte à côte.
